# EUROPÄISCHE PATENTANMELDUNG

(11) **EP 1 155 656 A1**
(43) Veröffentlichungstag der Anmeldung: **21.11.2001**
(21) Anmeldenummer: 01112294.2
(22) Anmeldetag: 18.05.2001
(51) Int. Cl.: A61B 3/024

(54) **Vorrichtung und Verfahren zur Gesichtsfelduntersuchung**

(30) Priorität: 18.05.2000 DE 10024509
(71) Anmelder: Löw, Bernhard, Dr. med., 66333 Völklingen (DE)
(72) Erfinder: Löw, Bernhard, Dr. med., 66333 Völklingen (DE)
(74) Vertreter: Hering, Hartmut, Dipl.-Ing.

(57) **Zusammenfassung**

Eine Vorrichtung zum Einsatz bei einer Gesichtsfelduntersuchung bzw. einer Perimetrie weist eine Lichtring-Erzeugungseinrichtung (10,11,12)zum Erzeugen eines Lichtrings, der von einem Patienten zu sehen ist; eine Lichtring-Änderungseinrichtung (12,14) zum Ändern des Lichtrings; und eine Lichtring-Darstellungseinrichtung (18) zum Darstellen des Lichtrings an einer Stelle auf, die im Gesichtsfeld eines Probanden liegt. Bei einem Verfahren zur Durchführung einer Gesichtsfelduntersuchung unter Verwendung der angegebenen Vorrichtung wird der erzeugte und vom Probanden erkennbare Lichtring bei sich änderndem Durchmesser in kurzer Zeit über das gesamte Gesichtsfeld bewegt, während der Proband seinen Blick auf das markierte Zentrum des Lichtrings richtet, wobei ein Gesichtsfeldausfall dann gegeben ist, wenn der Proband keinen geschlossenen sondern einen an einer oder mehreren Stellen offenen Lichtring sieht.

## Beschreibung

Die Erfindung betrifft eine Vorrichtung zur Gesichtsfelduntersuchung und ein Verfahren zur Verwendung derselben.

Die Sehleistung des Menschen ist im wesentlichen abhängig von der zentralen Sehschärfe und vom Gesichtsfeld. Von Augenärzten wird eine Gesichtsfelduntersuchung oder Perimetrie bei Patienten nicht routinemäßig durchgeführt, sondern nur dann, wenn Patienten über richtungsweisende Symptome klagen. Daher bleiben Gesichtsfelddefekte oft unentdeckt.

Jedoch bei einem konkreten Verdacht auf einen derartigen Defekt wird eine Untersuchung durchgeführt. Diese Untersuchung stellte bislang sowohl an den Untersucher als auch an den Patienten hohe Ansprüche. Sie kann beispielsweise manuell am mechanischen Perimeter, wie beispielsweise dem bekannten Goldmann-Gerät, durchgeführt werden.

Es gibt auch programmgesteuerte automatische Perimeter, die inzwischen derart weit verbreitet sind, daß fast alle Augenarztpraxen in Deutschland damit ausgestattet sind. Aber trotz aufwendiger Technik ist die Perimetrie nach wie vor zeitraubend und belastend. Beispielsweise kann es vorkommen, daß es bis zur Darstellung eines perimetrischen Defekts für ein Auge etwa 15 Minuten dauert und ein Patient während dieser Zeitdauer bis zu etwa 500 mal auf ihm gezeigte Änderungen reagieren muß. Dieses Beispiel läßt erkennen, daß es unmöglich ist, eine solche Untersuchung routinemäßig durchzuführen.

Insbesondere gibt es die Schwierigkeit, daß es für eine orientierende Prüfung sowohl des parazentralen als auch des peripheren Gesichtsfelds immer noch keinen geeigneten "Suchtest" oder "Übersichtstest", ein sogenanntes Screening-Verfahren, gibt.

Zur Ermittlung von absoluten und großflächigen Ausfällen behilft man sich im allgemeinen mit der sogenannten Konfrontationsmethode. Hierbei sitzen sich Untersucher und Patient gegenüber. Der Untersucher hält seine Hand im peripheren Gesichtsfeld des Patienten zunächst ruhig und bewegt dann nach unterschiedlich langer Wartezeit einen Finger. Der Patient muß dann mitteilen, daß er eine Bewegung erkennt. Diese Methode ist mit vielen Unsicherheiten belastet.

1988 haben Aulhorn und Mitarbeiter ein Screening-Verfahren entwickelt, bei dem Patienten auf einem Monitor ein Muster von kleinen Punkten dargeboten wird. Dieses Muster ist ähnlich einem Fernsehbild nach Sendeschluß. Bei dieser sogenannten Rauschfeld-Kampimetrie muß der Patient seinen Blick auf eine auf dem Monitor dargestellte zentrale farblich oder graphisch markierte Stelle richten und soll dann eventuell vorhandene Defekte mit dem Finger auf dem Bildschirm anzeigen. Das bedeutet, daß der Patient eine Markierung auf dem Bildschirm oder Monitor fixieren soll und während der gesamten Untersuchung den Blick nicht von dieser Stelle abwenden darf. Zugleich soll er aber seine Aufmerksamkeit auf eine Stelle lenken, an der er kein Rauschen bemerkt, und diese Stelle mit dem Finger auf dem Bildschirm zeigen oder umfahren.

Dieses Screening-Verfahren hat den Nachteil, daß die bei dieser Untersuchung dem Patienten zugeteilte Aufgabe, zentral zu fixieren und gleichzeitig eventuell vorhandene Defekte mit dem Finger im parazentralen und peripheren Gesichtsfeld nachzuzeichnen, eine Trennung der gewohnten Verbindung von Wahrnehmung und Motorik voraussetzt. Weil die zum Nachzeichnen erforderliche motorische Steuerung der Muskulatur nicht automatisch, d.h. nicht als komplexes, vorprogrammiertes Bewegungsmuster abläuft, kann die Untersuchung nicht besonders gut funktionieren. Kurz gesagt, kann ein Patient bei Anwendung des angegebenen Untersuchungsverfahrens vollkommen überfordert werden.

Angesichts des vorangehend beschriebenen Standes der Technik ist es eine Aufgabe der Erfindung, eine Vorrichtung zur Gesichtsfelduntersuchung zu schaffen, die einfach aufgebaut ist und auf einfache Weise zu bedienen ist. Weiterhin soll ein Verfahren geschaffen werden, bei dem die Vorrichtung gemäß der Erfindung verwendet wird.

Diese Aufgabe wird erfindungsgemäß durch die im Anspruch 1 angegebene Vorrichtung gelöst. Die abhängigen Ansprüche 2 bis 8 zeigen spezielle Ausführungsformen der Vorrichtung gemäß Anspruch 1. Im Anspruch 9 ist ein erfindungsgemäßes Verfahren angegeben, das unter Verwendung der erfindungsgemäßen Vorrichtung durchgeführt wird.

Die erfindungsgemäße Vorrichtung zur Gesichtsfelduntersuchung ist einfach aufgebaut und kann auf einfache Weise bedient werden. Ein erfindungsgemäßes Verfahren zur Perimetrie unter Verwednung der erfindungsgemäßen Vorrichtung hat die Vorteile, daß eine orientierende Gesichtsfelduntersuchung einfacher, sicherer und schneller als im Stand der Technik durchgeführt werden kann.

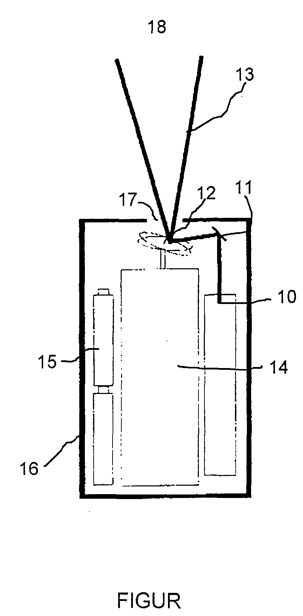

Die angegebenen und weitere Merkmale und Einzelheiten der Erfindung werden einem Fachmann auf dem Gebiet aus der folgenden detaillierten Beschreibung und der beigefügten Zeichnung klarer, die Merkmale der vorliegenden Erfindung anhand eines Beispiels darstellt und wobei die
- Figur: eine Seitenansicht einer bevorzugten Ausführungsform der erfindungsgemäßen Vorrichtung zum Einsatz bei einer Gesichtsfelduntersuchung der Erfindung zeigt.

Die erfindungsgemäße Vorrichtung zur Gesichtsfelduntersuchung weist eine Lichtring-Erzeugungseinrichtung 10, 11, 12, eine Lichtring-Änderungseinrichtung 12, 14 und eine Lichtring-Darstellungseinrichtung 18 auf. Die Lichtring-Änderungseinrichtung 12, 14 kann den von der Lichtring-Erzeugungseinrichtung 10, 11, 12 erzeugten Lichtring in bezug auf den Durchmesser, die Breite, die Farbe und die Leuchtdichte ändern.

Insbesondere sind als Vorrichtung zum Einsatz bei einer Gesichtsfelduntersuchung Diaprojektoren geeignet, sowie auch Video- oder Datenprojektoren.

Als Lichtring-Erzeugungseinrichtung können Lichtquellen an einem Stab angebracht sein, der in Rotation versetzt wird. Wenn dann die Rotationsfrequenz über der Bildverschmelzungsfrequenz des Auges eines Betrachters liegt, entsteht für den Betrachter der Eindruck eines geschlossenen Lichtrings. Die Lichtquellen können auch auf einer Scheibe angebracht sein, welche in Rotation versetzt wird. Zur Änderung des Lichtrings können die Lichtquellen bei den vorangehend angegebenen Beispielen verschiebbar angebracht sein.

Es ist auch möglich, Lichtringe in einem Rechner zu erzeugen und auf einem Computermonitor darzustellen. Bei diesem Beispiel kann eine Änderung der Lichtringe programmgesteuert erfolgen.

Eine bevorzugte Vorrichtung zur Gesichtsfelduntersuchung ist in der Figur gezeigt. Dabei besteht die Lichtring-Erzeugungseinrichtung aus einer oder mehreren Laserdioden 10, einem Umlenkspiegel 11 und einem weiteren Umlenkspiegel 12, der drehbar und in seiner Neigung variabel ist. Dieser weitere Umlenkspiegel 12 bildet beim bevorzugten Ausführungsbeispiel der Figur zusammen mit einem Motor 14 zum Antreiben des weiteren Umlenkspiegels 12 die Lichtring-Änderungseinrichtung. Weiterhin sind in der Figur Batterien 15 zum Versorgen der Laserdioden 10 und des Motors 14 mit Strom und ein von der Laserdiode 10 kommender Laserstrahl 13 gezeigt. Die einzelnen Bauteile sind von einem Gehäuse 16 umgeben, das an einer Seite eine Öffnung 17 zum Durchlassen des Laserstrahls 13 aufweist.

Unter Bezugnahme auf die Figur wird nun die Funktion der gezeigten Vorrichtung zur Gesichtsfelduntersuchung detaillierter erklärt.

Der von der oder den Laserdioden 10 kommende Laserstrahl 13 wird durch den Umlenkspiegel 11 in Richtung zum weiteren Umlenkspiegel 12 abgelenkt, von wo er als Lichtring durch die Öffnung 17 des Gehäuses nach außen auf eine für eine Untersuchung geeignete Fläche 18, die die Lichtring-Darstellungseinrichtung ist, abgegeben wird. Der weitere Umlenkspiegel 12 erzeugt den Lichtring, indem er angetrieben durch den Motor 14 in geneigter Stellung gedreht wird. Durch Änderung der Neigung des weiteren Umlenkspiegels 12 entstehen unterschiedliche Kreis- oder Ringdurchmesser des Lichtrings. Der Lichtring kann somit auf einfache Weise kontinuierlich verkleinert oder vergrößert werden. Dabei kann innerhalb kurzer Zeit das gesamte Gesichtsfeld überstrichen werden.

Im folgenden wird das erfindungsgemäße Verfahren unter Verwendung der Vorrichtung zur Gesichtsfelduntersuchung genauer beschrieben.

Einem Patienten wird ein durch die Vorrichtung zur Gesichtsfelduntersuchung erzeugter Lichtring oder Lichtkreis gezeigt. Dieser Lichtring wird dann bei sich änderndem Durchmesser in kurzer Zeit über das gesamte Gesichtsfeld bewegt. Das Zentrum bzw. der Mittelpunkt des Lichtkreises ist als Fixationspunkt markiert. Hierzu kann man beispielsweise einen zweiten Laserstrahl benutzen, der das Gesichtsfeldzentrum graphisch oder farblich markiert. Hält der Patient seinen Blick auf diesen Punkt fixiert, projiziert sich der Lichtring auf die zu untersuchenden Netzhautbereiche. Sind diese intakt, bzw. werden deren Nervenpunkte im Gehirn physiologisch richtig verarbeitet, dann erkennt der Patient einen geschlossenen und einheitlichen Ring. Sollte der Ring dem Patienten jedoch an einer oder an mehreren Stellen offen bzw. unterbrochen erscheinen, ist dies ein sicherer Hinweis für das Vorliegen eines vollständigen Defekts des Gesichtsfelds.

Beim Einsatz des erfindungsgemäßen Verfahrens unter Verwendung der Vorrichtung zur Gesichtsfelduntersuchung besteht für einen Patienten gegenüber dem Stand der Technik demgemäß die Anforderung in bezug auf die Sensorik in einem Erkennen, ob der gezeigte Lichtring geschlossen ist, und beschränkt sich die Anforderung in bezug auf die Motorik auf die Artikulation der Worte "ja" oder "nein" bzw. ein Nicken oder ein Schütteln des Kopfes.

Durch die erfindungsgemäße Vorrichtung und das erfindungsgemäße Verfahren können insbesondere die folgenden Vorteile erzielt werden:

Gemäß der Erfindung muß eine Untersuchung nicht mehr mit einzelnen Testmarken in einem bestimmten Sektor erfolgen, sondern sie erfolgt mit einem Licht, das sich über 360° erstreckt, wobei sich die Reize zur Blickrichtungsänderung gegenseitig aufheben. Somit wird der Patient in geringerem Ausmaß zur reflektorischen Blickwendung verleitet, weshalb die Fixierung des Blicks auf den Gesichtsmittelpunkt besser als beim Stand der Technik funktioniert.

Das Entscheidungskriterium, nämlich "ja" oder "nein", ist einfach und stellt keine hohen Anforderungen an einen Patienten.

Auftretende Defekte sind gut zu lokalisieren, wenn der Kreis mit dem Zifferblatt einer Uhr verglichen wird. Als Beispiel zeigt sich der Ausfall des Gesichtsfelds eines Patienten bei der orientierenden Perimetrie mittels eines Lichtrings im Bereich von 20° als Öffnung des Rings von 11°° bis 3°°.

Die zur Untersuchung erforderliche Vorrichtung erfordert keinen oder nur einen geringen technischen Aufwand und ist transportierbar. Weiterhin ist die Untersuchung nicht zeitaufwendig.

Die vorliegende Erfindung zeigt eine Vorrichtung und ein Verfahren zur Gesichtsfelduntersuchung. Vorteilhafterweise ist die Vorrichtung einfach aufgebaut und kann auf einfache Weise bedient werden. Eine mittels der Vorrichtung gemäß der Erfindung durchgeführte Gesichtsfelduntersuchung bzw. Perimetrie verlangt von einem Patienten keine schwierigen Aufgaben zu lösen und ist für einen Untersucher einfacher und schneller durchzuführen als im Stand der Technik.

## Patentansprüche

1. Vorrichtung zur Gesichtsfelduntersuchung, welche folgendes aufweist:
eine Lichtring-Erzeugungseinrichtung (10, 11, 12) zum Erzeugen eines Lichtrings, der von einem Patienten zu sehen ist;
eine Lichtring-Änderungseinrichtung (12, 14) zum Ändern des Lichtrings; und
eine Lichtring-Darstellungseinrichtung (18) zum Darstellen des Lichtrings an einer Stelle im Gesichtsfeld eines Probanden.

2. Vorrichtung nach Anspruch 1, **dadurch gekennzeichnet, daß** die Lichtring-Änderungseinrichtung den von der Lichtring-Erzeugungseinrichtung erzeugten Lichtring in bezug auf den Durchmesser, die Breite, die Farbe und die Leuchtdichte ändert.

3. Vorrichtung nach Anspruch 1 oder 2, **dadurch gekennzeichnet, daß** als Lichtring-Erzeugungseinrichtung und Lichtring-Änderungseinrichtung Diaprojektoren vorgesehen sind, sowie auch Video-oder Datenprojektoren.

4. Vorrichtung nach Anspruch 1 oder 2, **dadurch gekennzeichnet, daß** die Lichtring-Erzeugungseinrichtung Lichtquellen aufweist, die an einem Stab oder auf einer Scheibe angebracht sind, der bzw. die in eine derartige Rotation versetzt wird, daß die Rotationsfrequenz über der Bildverschmelzungsfrequenz des menschlichen Auges liegt.

5. Vorrichtung nach Anspruch 1 oder 2, **dadurch gekennzeichnet, daß** als Lichtring-Erzeugungseinrichtung und Lichtring-Änderungseinrichtung ein Rechner vorgesehen ist und als Lichtring-Darstellungseinrichtung ein Computermonitor vorgesehen ist, wobei die Erzeugung, die Änderung und die Darstellung des Lichtrings programmgesteuert erfogt.

6. Vorrichtung nach Anspruch 1 oder 2, **dadurch gekennzeichnet, daß** die Lichtring-Erzeugungseinrichtung eine oder mehrere Laserdioden (10) aufweist, einen Umlenkspiegel (11) und einen weiteren Umlenkspiegel (12), der drehbar und in seiner Neigung variabel ist, und die Lichtring-Änderungseinrichtung den weiteren Umlenkspiegel (12) und einen Motor (14) zum Antreiben des weiteren Umlenkspiegel (12) aufweist.

7. Vorrichtung nach Anspruch 6, **dadurch gekennzeichnet, daß** Batterien (15) zur Stromversorgung der Laserdioden (10) und des Motors (14) vorgesehen sind.

8. Vorrichtung nach Anspruch 6 oder 7, **dadurch gekennzeichnet, daß** die Vorrichtung von einem Gehäuse (16) umgeben ist, das an einer Seite eine Öffnung (17) zum Durchlassen von Laserlicht (13) aufweist.

9. Verfahren zum Durchführen einer Gesichtsfelduntersuchung unter Verwendung der Vorrichtung nach einem der vorangehenden Ansprüche, **dadurch gekennzeichnet, daß** der erzeugte und im Gesichtsfeld des Probanden liegende Lichtring bei sich änderndem Durchmesser in kurzer Zeit über das gesamte Gesichtsfeld bewegt wird, während der Proband seinen Blick auf das markierte Zentrum des Lichtrings richtet, wobei ein Gesichtsfeldausfall dann gegeben ist, wenn der Proband keinen geschlossenen sondern einen an einer oder mehreren Stellen offenen Lichtring sieht.
